# EUROPEAN PATENT APPLICATION

(11) **EP 1 839 650 A1**
(43) Date of publication of application: **03.10.2007**
(21) Application number: 05822779.4
(22) Date of filing: 27.12.2005
(51) Int. Cl.: A61K 9/20, A61K 47/32

(54) **QUICK DISINTEGRATION TABLET AND METHOD OF PRODUCING THE SAME**

(30) Priority: 28.12.2004 JP 2004381742
(71) Applicant: Eisai R&D Management Co., Ltd., Tokyo 112-8088 (JP)
(72) Inventor: SUZUKI, Toshio, EISAI CO., LTD., Honjo-shi, Saitama 367-0048 (JP)
(74) Representative: HOFFMANN EITLE
(86) International application number: PCT/JP2005/023985
(87) International publication number: WO 2006/070845

(57) **Abstract**

To provide a production method of a rapidly disintegrating tablet, the method that has versatility and, without a specialized device, can efficiently produce rapidly disintegrating tablets having a desired rapid disintegration property and sufficient strength. And also to provide such rapidly disintegrating tablets produced by the method, the tablets that can be easily swallowed by people such as the elderly, children and patients having a poor swallowing capability and that have sufficient strength, so that they can endure breakage and abrasion in processes such as distribution and storage and dispensing processes by tablet packing machines. The method of such rapidly disintegrating tablet includes: (1) mixing an active ingredient, an acrylic copolymer and at least a pharmaceutically acceptable additive to obtain a mixture thereof (2) tableting the mixture to obtain a compact, and (3) isothermally heating the compact at a temperature of 50°C to 100°C for a given period of time. And also to provide such rapidly disintegrating tablets produced by the production method of a rapidly disintegrating tablet.

## Description

### Technical Field

The present invention relates to a rapidly disintegrating tablet and a production method thereof, and more particularly relates to a rapidly disintegrating tablet having high tablet strength and a production method thereof.

### Background Art

Tablets are frequently used as a dosage form of therapeutic agents for their convenience and the easiness with which they are swallowed.

As tablets are formed into particular shapes, their breakage and abrasion during distribution need to be prevented. Thus, not only disintegration property of the tablets and dissolution property of drug but also their strength needs to be taken into consideration. In recent years, tablets that are developed for children and elderly people who have poor swallowing capability so that they can easily disintegrate in the oral cavity without water are required to be as strong as general tablets. Moreover, such tablets need to be strong enough not only to endure breakage and abrasion during distribution processes but also to be used in auto tablet packing machines which have been introduced for use in medical preparation processes for packaging two or more pills into a single package.

For those reasons, specialized tableting machines and production methods of tablets designed for producing rapidly disintegrating tablets with improved strength and abrasion resistance have been developed. For example, Patent Literature 1 discloses a production method of a tablet with increased strength, the method including (a) a process whereby a water-soluble meltable binder, at least a filler, and an active pharmaceutical ingredient are combined and compressed into a tablet (b) a process whereby the water-soluble meltable binder is melted in the tablet, and (c) a process whereby the water-soluble meltable binder is solidified.

In the disclosed method, the melting point of the water-soluble meltable binder is in the range of 38°C to 62°C. Thus, when tablets are exposed to a temperature equal to or above their melting point during, for example, a distribution process, the water-soluble meltable binder may be melted, resulting in change in its shape and property. Additionally, when exposed to a high-humidity environment, the tablet may be melted, resulting in reduction in their strength and/ or occurrence of mutual adhesion of the tablets.

On the other hand, Patent Literature 2 proposes an orally disintegrating tablet and a production method thereof. This orally disintegrating tablet contains a drug, a diluent, saccharides having lower melting points than the drug and diluent, and a binder. The saccharides are uniformly formulated into the tablet, and melt-solidified products of the saccharides are used for cross-linking particles of the drug and/ or diluent to thereby form the tablet. However, as the tablet disclosed in Patent Literature 2 uses the saccharides as a cross-linking agent, the saccharides may be melted when the tablet is stored in a high-humidity environment. It will result in a reduction in strength and/or occurrence of mutual adhesion of the tablets.

Thus, there is a demand for a production method of a rapidly disintegrating tablet, the method that has versatility and, without a specialized device, can efficiently produce a rapidly disintegrating tablet having a desired rapid disintegration ability and sufficient strength. And there is also a demand for such rapidly disintegrating tablets produced by the method, the tablets that can be easily swallowed by people such as the elderly, children and patients having a poor swallowing capability and that have sufficient strength, so that they can endure breakage and abrasion during processes such as distribution and storage, and medical preparation processes to make one dose pack with automatic packaging machines for dispensing medicine.
Patent Literature 1: Japanese Patent Application Publication (JP-B) No. 2640570
Patent Literature 2: Japanese Patent (JP-B) No. 2004-43472

### Disclosure of Invention

An object of the present invention is to solve the forementioned problems and to achieve the following objects: that is, to provide a production method of a rapidly disintegrating tablet, the method that has versatility and, without a specialized device, can efficiently produce a rapidly disintegrating tablet having desired rapid disintegration ability and sufficient strength. And also to provide such rapidly disintegrating tablets produced by the method, the tablet that can be easily swallowed by people such as elderly, children and patients having a poor swallowing capability and has sufficient strength so that it can endure breakage and abrasion in processes such as distribution and storage and a dispensing process by a tablet packing machine.

The inventors of the present invention conducted studies in order to solve the forementioned problems and established that a rapidly disintegrating tablet having rapid disintegration ability while maintaining its strength can be obtained by mixing an acrylic copolymer and at least a pharmaceutically acceptable additive, subjecting the mixture thus obtained to tableting and isothermally heating the compact thus obtained to a temperature equal to or above the glass transition temperature of the acrylic copolymer, so that at least a part or the entire of the acrylic copolymer is cross-linked to form stabilized structure in the tablet.

The strength of a rapidly disintegrating tablet can be given based on the hardness and/ or abrasion resistance of the tablet, the strength being usually proportional to hardness. Thus, in the following descriptions, the term "strength" of the rapidly disintegrating tablet of the present invention shall be used as synonymous with the term "hardness."

Additionally, the "rapidly disintegrating tablet" in the present invention shall mean a tablet that disintegrates with a small amount of water after it is administrated or swallowed. Particularly, the "orally disintegrating tablet" in the present invention shall mean a tablet that can completely disintegrate in the oral cavity within 2 minutes only by saliva without water.

The present invention is based on the above-stated knowledge of the inventors, and means to solve the above-stated problems are as follows:
<1> A production method of a rapidly disintegrating tablet, including:
   (1) mixing an active ingredient, an acrylic copolymer and at least a pharmaceutically acceptable additive to obtain a mixture thereof,
   (2) tableting the mixture to obtain a compact of the mixture, and
   (3) isothermally heating the compact at a temperature in the range of from 50°C to 100°C for a given period of time.
<2> The production method according to <1>,
   wherein the active ingredient is a medicinal ingredient.
<3> The production method according to one of <1> and <2>,
   wherein
   the compact is isothermally heated at a temperature equal to or above the glass-transition temperature (Tg) of the acrylic copolymer for a given period of time.
<4> The production method according to any one of <1> to <3>,
   wherein
   the acrylic copolymer is composed of a methyl methacrylate, a butyl methacrylate and a dimethylaminoethyl methacrylate.
<5> The production method according to any one of <1> to <4>,
   wherein
   the acrylic copolymer is aminoalkyl methacrylate copolymer E.
<6> The production method according to any one of <1> to <5>,
   wherein
   the formulating amount of the acrylic copolymer is in the range of 0.5 parts by mass to 20 parts by mass per 100 parts by mass of the compact.
<7> The production method according to any one of <1> to <6>,
   wherein
   the formulating amount of the acrylic copolymer is in the range of 0.5 parts by mass to 9 parts by mass per 100 parts by mass of the compact.
<8> The production method according to any one of <1> to <7>,
   wherein
   the formulating amount of the acrylic copolymer is in the range of 1 part by mass to 6 parts by mass per 100 parts by mass of the compact.
<9> The production method according to any one of <1> to <8>,
   wherein
   the formulating amount of the acrylic copolymer is in the range of 2 parts by mass to 4 parts by mass per 100 parts by mass of the compact.
<10> The production method according to any one of <1> to <9 >, wherein
   the average particle diameter of the acrylic copolymer is in the range of 1µm to 500µm.
<11> The production method according to any one of <1> to <10>, wherein
   the compact is isothermally heated at a temperature in the range of from 70°C to 90°C for a given period of time.
<12> A production method of the base for a rapidly disintegrating table, including (1) mixing an active ingredient, an acrylic copolymer and at least a pharmaceutically acceptable additive to obtain a mixture thereof,
   (2) tableting the mixture to obtain a compact of the mixture, and
   (3) isothermally heating the compact at a temperature in the range of from 50°C to 100°C for a given period of time.
<13> The production method according to <12>, wherein
   the compact is isothermally heated at a temperature equal to or above the glass-transition temperature (Tg) of the acrylic copolymer for a given period of time.
<14> The production method according to one of <12> and <13>,
   wherein
   the acrylic copolymer is composed of a methyl methacrylate, a butyl methacrylate and a dimethylaminoethyl methacrylate.
<15> The production method according to any one of <12> to <14>, wherein the acrylic copolymer is aminoalkyl methacrylate copolymer E.
<16> The production method according to any one of <12> to <15>, wherein the formulating amount of the acrylic copolymer is in the range of 0.5 parts by mass to 20 parts by mass per 100 parts by mass of the compact.
<17> The production method according to any one of <12> to <16>, wherein the formulating amount of the acrylic copolymer is in the range of 0.5 parts by mass to 9 parts by mass per 100 parts by mass of the compact.
<18> The production method according to any one of <12> to <17>, wherein the formulating amount of the acrylic copolymer is in the range of 1 part by mass to 6 parts by mass per 100 parts by mass of the compact.
<19> The production method according to any one of <12> to <18>, wherein the formulating amount of the acrylic copolymer is in the range of 2.0 parts by mass to 4.0 parts by mass per 100 parts by mass of the compact.
<20> The production method according to any one of <12> to <19 >, wherein the average particle diameter of the acrylic copolymer is in the range of 1µm to 500µm.
<21> The production method according to any one of <12> to <20>, wherein the compact is isothermally heated at a temperature in the range of from 70°C to 90°C.
<22> A rapidly disintegrating tablet which is produced by the production method of a rapidly disintegrating tablet according to any one of <1> to <11>.
<23> A rapidly disintegrating tablet, wherein
   the rapidly disintegrating tablet is produced using the base produced by the production method of a base for a rapidly disintegrating tablet according to any one of <12> to <21>.
<24> The rapidly disintegrating tablet according to one of <22> and <23>, wherein the hardness of the rapidly disintegrating tablet is 1.1 to 10 times higher than the hardness of the compact that is to be isothermally heated at a temperature in the range of 50°C to 100°C.
<25> The production method according to any one of <22> to <24>, wherein the rapidly disintegrating tablet is a plain tablet, an effervescent tablet or an orally disintegrating tablet.

According to the present invention, it is possible to solve the conventional problems, and can produce a rapidly disintegrating tablet having desired rapid disintegration ability and sufficient strength, and can provide a production method of a rapidly disintegrating tablet, the method that has versatility and can efficiently produce the tablet. And the present invention can further provide such rapidly disintegrating tablets produced by the method, the tablet that can be easily swallowed by people having swallowing difficulty and that have sufficient strength so that they can endure breakage and abrasion in processes such as distribution and storage and a dispensing process by a tablet packing machine. Moreover, the production method can be used for production methods of tablets for, for example, food and cosmetic purposes.

### Brief Description of Drawings

FIG.1 is a flowchart of the production method of the present invention.
FIG. 2 shows the result of Example 1 which investigated the effect of an isothermal heating process and tableting pressure on the hardness of an orally disintegrating tablet during a tableting process in the present invention.
FIG. 3 shows the result of the dissolution test of the orally disintegrating tablets of Examples 2 and 3.
FIG. 4 shows the result of Example 7 which investigated the effect of isothermal holding time in an isothermal heating process on the hardness of an orally disintegrating tablet.
FIG. 5 shows the result of Example 7 which investigated the effect of isothermal holding time in an isothermal heating process on the dissolution test of an orally disintegrating tablet.

### Best Mode for Carrying Out the Invention

These and other objects of the present invention will become more apparent in the following embodiments. It is to be expressly understood, however, that the embodiments are for purpose of illustration only and are not intended as a definition of the limits of the invention. Various changes in the form of carrying out the invention, within the scope of the claims, may be made.

### (Production Method of Rapidly Disintegrating Tablet and of Base for the Tablet)

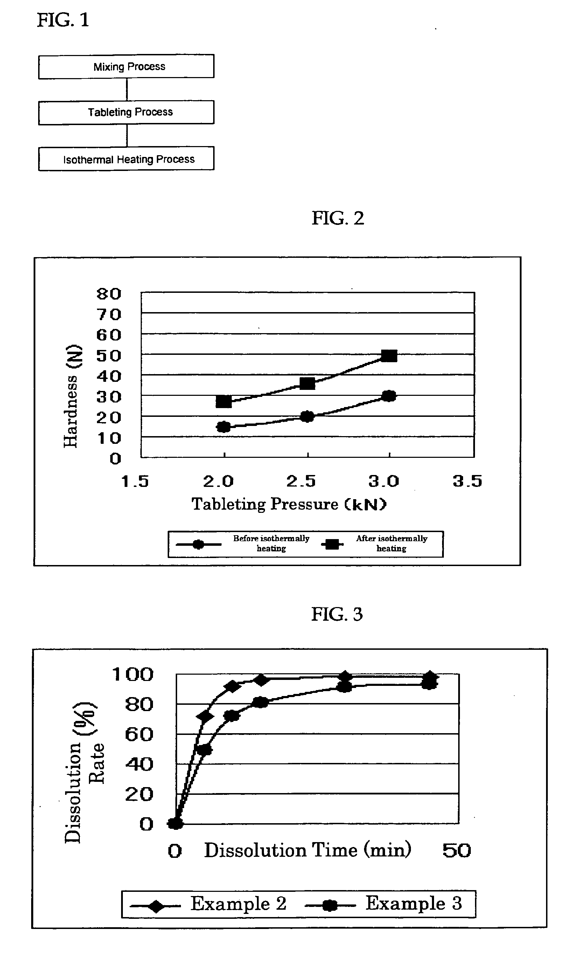

The production methods of the rapidly disintegrating tablet of the present invention and the base for the tablet include (1) a raw material mixing process (2) a tableting process in which a mixture obtained in the raw material mixing process is tableted, and (3) an isothermal heating process. And the production methods may further contain other processes such as a granulation process, a granulating process and/ or a coating process upon necessity. An embodiment of the present invention is shown in FIG. 1.

The raw material used in the production method of a rapidly disintegrating tablet is composed of an active ingredient, an acrylic copolymer and at least a pharmaceutically acceptable additive. Using the raw material and the production method of a rapidly disintegrating tablet, rapidly disintegrating tablets for medical, quasi drug, food and cosmetic purposes can be obtained according to a contained active ingredient.

The raw material used in the production method of a base for a rapidly disintegrating tablet contains at least an acrylic copolymer and a pharmaceutically acceptable additive, and it may further contain other suitable components upon necessity. Using the raw material in the production method of a base for a rapidly disintegrating tablet, the base for rapidly disintegrating tablets can be obtained.
(1) Mixing Process
   The mixing process is a process for uniformly mixing the raw material and preparing a mixture.
   A method to uniformly mix the raw material is not particularly limited, and a suitable method can be selected from, for example, methods using mixers according to the purpose. Examples of the methods include mixing using a V blender (such as those manufactured by TOKUJU CORPORATION, Fuji Paudal Co. Ltd., and DALTON CORPORATION) at 5 rpm to 20 rpm, mixing using a tumbling mixer (such as those manufactured by DALTON CORPORATION and DAIKO SEIKI Co., Ltd.) at 2 rpm to 10 rpm, and mixing using a high-speed mixer (such as those manufactured by OKADA SEIKO CO., LTD., NARA MACHINERY CO., LTD., and POWREX Corporation) at 10 rpm to 200 rpm.
   Those mixers can be used alone or in combination.
   The raw material may be pulverized before the mixing process.
   Moreover, one or more known granulation operations and/ or granulating operations may be conducted to the drug to obtain granulated particles. Examples of the granulation operations include dry granulation, wet granulation and melt cooling granulation. Additives such as vehicles and binders may be used in the operations in accordance with necessity. The granulated particles can be used for the mixing process.
(2) Tableting Process
   The tableting process is a process for tableting the mixture obtained in the (1) mixing process.
   A method to tablet the mixture is not particularly limited, and a suitable method can be selected from, for example, methods using tableting machines. Examples of the methods include methods using a single stroke tableting machine, a rotary tableting machine (such as those manufactured by HATA IRON WORKS CO., LTD. and KIKUSUI SEISAKUSHO LTD.) and a dry-coat tableting machine.
   A tableting pressure in the tableting process is, for example, in the range of 1.0kN to 20kN. That pressure is preferably in the range of 1.2kN to 15kN, and more preferably 1.5kN to 12kN.
(3) Isothermal Heating Process
   The isothermal heating process is a process for keeping the compact obtained in the (2) tableting process at a temperature in the range of 50°C to 100°C for a given period of time.

The method for isothermally heating the compact at a temperature in the range of 50°C to 100°C for a given period of time is not particularly limited, and, according to the purpose, a suitable method can be selected from, for example, methods using known heat retention devices to isothermally heating an object for a given period of time. Examples of such method include keeping the compact in, for example, a constant temperature bath or a shelf dryer for a given period of time. They are equipped with a warming means such as warm air, infrared rays and a halogen lamp. Additionally, a method in which the isothermal heating process is continuously conducted after the tableting process may be used. In this method, a heat retention device (such as an isothermal heating chamber), located on a production line following to a tableting machine for the tableting process, will be used.

An isothermal temperature in the isothermal heating process is in the range of 50°C to 100°C. The temperature is not particularly limited and can be set at a suitable level, provided the temperature is equal to or higher than the glass transition temperature (Tg) of the acrylic copolymer, but it is preferably a temperature at which the active ingredient of, for example, drug formulated as raw material will not be decomposed.

For example, when aminoalkyl methacrylate copolymer E which has a glass transition temperature of about 50°C is used as the acrylic copolymer, the isothermal temperature in the isothermal heating process is preferably 50°C or higher, and more preferably in the range of 70°C to 90°C.

By isothermally heating the acrylic copolymer at temperature equal to or higher than the glass transition temperature thereof for a given period of time, the acrylic copolymer is cross-linked in the compact. As a result, a rapidly disintegrating tablet that has high hardness and is excellent in strength can be obtained.

By adjusting an isothermal holding time and temperature, the degree of the cross linking can be controlled. And further, by adjusting the content of the acrylic copolymer, the rapid disintegration ability and strength of the resulting rapidly disintegrating tablet can be controlled.

By suitably adjusting the balance between these three factors - the isothermal holding time and temperature in the isothermal heating process and the content of the acrylic copolymer - the rapid disintegration ability of the rapidly disintegrating tablet can be achieved at a desirable level while maintaining its strength.

The isothermal holding time in the isothermal heating process is not particularly limited and can be adjusted according to targeted rapid disintegration ability and strength of a rapidly disintegrating tablet.

For example, when the isothermal temperature is in the range of 70°C to 90°C, the length is preferably in the range of 0.2 to 48 hours, more preferably 0.5 to 24 hours, and further preferably 1 to 18 hours.

After completion of the isothermal heating process, it may be allowed to stand and cool to normal temperature (room temperature) by cutting the heat source of the above-stated heat retention device, or it may be cooled down to a desired temperature with a cooling device.

### <Raw Material>

### - Acrylic Copolymer -

The formulating amount of the acrylic copolymer is preferably in the range of 0.5 parts by mass to 20 parts by mass per 100 parts by mass of the compact obtained by mixing and tableting the raw material. That amount is more preferably in the range of 0.5 parts by mass to 9 parts by mass, particularly preferably 1 part by mass to 6 parts by mass, and most preferably 2 parts by mass to 4 parts by mass per 100 parts by mass of the compact.

When the formulating amount of the acrylic copolymer is more than 20 parts by mass per 100 parts by mass of the compact, the disintegration time of the rapidly disintegrating tablet may be elongated.

Preferred examples of the acrylic copolymer include copolymers composed of methyl methacrylate, butyl methacrylate and dimethylaminoethyl methacrylate, and aminoalkyl methacrylate copolymer E, a cationic methacrylate copolymer, is more preferable.

Aminoalkyl methacrylate copolymer E is a compound which is usually used as a coating agent. Preferred examples of aminoalkyl methacrylate copolymer E include a granulated type (tradename: EUDRAGIT (registered trademark) E100, manufactured by Degussa / imported and distributed by Higuchi, Inc.) and a fine particle type (tradename: EUDRAGIT (registered trademark) EPO, manufactured by Degussa / imported and distributed by Higuchi, Inc.).

In the mixing process, aminoalkyl methacrylate copolymer E may be added as powder, or dispersed into water or organic solvent to be used. Or a marketed organic solvent (tradename: EUDRAGITE 12.5, manufactured by Rohm) in which EUDRAGIT (registered trademark) E100 is dispersed may be used.

Alternatively, aminoalkyl methacrylate copolymer RS may be used as the acrylic copolymer.

The range of the average particle diameter of the acrylic copolymer is not particularly limited, and can be a suitable diameter according to the purpose. The average particle diameter is preferably in the range of 1µm to 500µm, more preferably 1µm to 300µm, further preferably 1µm to 50µm, and most preferably 3µm to 15µm.

### - Additive -

The additive is not particularly limited, provided it is pharmaceutically acceptable. Examples thereof include fillers, binders, lubricants, disintegrants, coating agents, plasticizers, suspensions (or emulsifiers), aromatizing agents, sugar-coating agents, moisture-proof agents, fluidizers and colorants. Examples of the additive are shown below; however, those examples are not intended to limit additives which can be used in the present invention. They can be used alone or in combination.

Examples of the fillers include D-mannitol; white sugars (including purified white sugars); sodium hydrogencarbonates; corn starch; potato starch; wheat starch; rice starch; partly alpha-ized starches; crystalline cellulose; light anhydrous silicic acids; phosphoric anhydrous; precipitated calcium carbonates; and calcium silicates.

Examples of the binders include povidones, dextrins, hydroxypropylcelluloses, hydroxypropyl methylcelluloses, methyl celluloses, polyvinyl alcohols, carboxymethylcellulose sodiums, pregelatinized starches, sodium alginates, pullulans and powdered acacia.

Examples of the lubricants include hardened oils, hydrogenated-castor oils, magnesium stearates, calcium stearates, glyceride behenates and sodium stearyl fumarates.

Examples of the disintegrants include low substituted hydroxypropylcelluloses, carmelloses, carboxy-methyl-starch sodiums and cross povidones.

Examples of the coating agents include cellulosics such as hydroxypropylcellulose, hydroxypropyl methylcellulose and carmellose sodium; synthetic macromolecules such as polyvinyl pyrrolidone, polyvinyl alcohol, and polyoxyethylene polyoxypropylene glycol; polysaccharides such as pullulan and chitosan; and natural high polymers such as gelatins, gum arabics, and shellacs.

Examples of the plasticizers include dioctyl adipate, triethyl citrate, triacetins, glycerols, concentrated glycerins and propylene glycol.

Examples of the suspensions (or emulsifiers) include lecithins; sucrose fatty acid esters; poly glycerine fatty acid esters; polyoxyethylene hardened castor oils; polysorbates; and copolymers of a polyoxyethylene and polyoxypropylene.

Examples of the aromatizing agents include menthols, peppermint oils, lemon oils and orange oils.

Examples of the sugar coating agents include white sugars, lactoses, starch syrups, precipitated calcium carbonates, gum arabics, carnauba waxes, shellacs, white beeswaxes and methyl celluloses.

Examples of the moisture proof agents include magnesium silicates, light anhydrous silicic acids, hydrogenated oils, magnesium stearates, paraffins, castor oils and shellacs.

Examples of the fluidizers include water silicon dioxides; light anhydrous silicic acids; heavy anhydrous silicic acids; crystalline celluloses; synthetic aluminum silicates; magnesium hydroxide-aluminiums; magnesium aluminometasilicates; stearic acids; calcium stearates; magnesium stearates; calcium tertiary phosphates; talcs; and corn starches.

Examples of the colorants include tar pigments such as Food Yellow No. 4, Food Yellow No. 5, Food Red No. 2, Food Red No. 102, Food Blue No.1, Food Blue No. 2 (indigo carmine), and Food Yellow No. 4 Aluminum Lake; titanium oxides; zinc oxides; and talcs.

### - Active Ingredient -

The active ingredient is not particularly limited, provided it can benefit when swallowed. A suitable active ingredient can be selected according to the purpose. Examples thereof include medicinal ingredients.

Examples of the medicinal ingredients include drugs.

### - Drug -

The drug is not particularly limited, and suitable one can be selected from, for example, antitumor drugs, antibiotics, anti-inflammatory drugs, analgesics, osteoporosis drugs, antihyperlipidemic drugs, antibacterial drugs, sedatives, ataraxics, antiepileptics, anti-depressants, remedies for alimentary diseases, anti-allergic disease drugs, anti-high-blood-pressure drugs, remedies for arteriosclerosis, diabetic drugs, hormone drugs and fat soluble vitamin drugs.

Examples of the antitumor drugs include danazol, busulfan, camptothecin, triiodo benzoate, taxol, doxorubicin hydrochloride, methotrexate, etoposide, 5-fluorouracil, mitoxantrone, mesna, dimesna, amino glutethimides, tamoxyphen, acroline, cisplatin, carboplatin and cyclophosphamides.

Examples of the antibiotics include amikacin and gentamycin.

Examples of the anti-inflammatory drugs include aspirin, phenacetin, acetamino phenon, phenylbutazon, keto phenylbutazon, mefenamic acid, bucolome, benzydamine, mepirizole, tiaramide, tinoridine; anti-inflammatory steroids such as prednisolone, hydrocortisone, methylprednisolone, dexa- betamethasone and betamethasone; indomethacin; diclofenac; loxoprofen; ibuprofen; and piroxicam.

Examples of the analgesics include xylocaine, pentazocine and aspirin.

Examples of the osteoporosis drugs include vitamin K2, prostaglandin A1, vitamin D, sex hormone derivatives, phenol sulfonic phthalein, benzothiopyran and thieno-indazoles.

Examples of the antihyperlipidemic drugs include clinofibrate, clofibrate, cholestyramine, soy sterols, tocopherol nicotinates, nicomol, niceritrol, probucol and elastase.

Examples of the ataraxics include benzodiazepines such as diazepam, lorazepam, and oxazolam.

Examples of the antiepileptics include phenytoin, phenobarbital, carbamazepine and primidone.

Examples of the anti-depressants include imipramine, noxiptiline and phenelzine.

Examples of the anti-dementia drugs include donepezil hydrochloride, galanthamine hydrobromic acid salts, rivastigmine tartrate, memantine hydrochloride and tacrin. Examples of anti-anxiety drugs include flurazepam hydrochlorides, alprazolam, tandospirone citrate and rilmazafone hydrochlorides.

Examples of the remedies for alimentary diseases include teprenone, metoclopramide, famotidine, sulpiride, trepibutone. Also include benzimidazole compounds having proton pump inhibitory action and potent inhibitory action on the gastric acid secretion and physiologically acceptable salts of the benzimidazole compounds. Examples of the benzimidazole compounds include omeprazole, esomeprazole, lansoprazole and pantoprazole.

Examples of the anti-allergic disease drugs include clemastine fumarate, cyproheptadine hydrochlorides, diphenhydramine, ramine-T method, clemizole and methoxyphenamine.

Examples of the anti-high-blood-pressure drugs include nicardipine hydrochloride, delapril hydrochlorides, captopril, prazosin hydrochloride and reserpine.

Examples of the remedies for arteriosclerosis include inhibitors of cholesterol ester transfer protein.

Examples of the diabetic drugs include glymidine, glipizide, glibenclamide, buformin and metformin.

Examples of the hormone drugs include dexamethasone, betamethasone, prednisolone, hydrocortisones, triamcinolone, triamcinolone acetonides, fluocinolone acetonides, hexestrols, methimazole and estriols.

Examples of the vitamin drugs include Vitamin A, vitamin B, vitamin C, vitamin D, vitamin E, a vitamin K and folic acid.

Examples of the herbal medicines include liliaceae, lycii fructus, bezoar bovis, dioscorea rhizome, paeonia lactiflora pall, rehmanniae radix, Agkistrodon Japonicae, coicis semen, longan arillus, eucommia ulmoides, cervi parvum cornu, royal jelly, a cinnamomi cortex, liquorice, ginseng and angelicae radix.

Examples of the medicinal ingredients other than drugs include quasi drugs, food for specified health use and nutritional performance food. Specific examples are drugs, such as vitamins, ubidecarenones and carnitines, having moderate effect; minerals such as zinc and iron; and amino acids such as aspartic acid and arginine.

### - Other Active Ingredients -

Examples of the active ingredient other than the medicinal ingredients include food ingredients and cosmetics ingredients.

The formulating amount of the active ingredient is not particularly limited and can be adjusted at a suitable level according to the purpose, provided the amount is in a range in which the rapid disintegration ability and strength of the rapidly disintegrating tablet are not degraded.

### (Rapidly Disintegrating Tablet)

The rapidly disintegrating tablet, produced by the production method of the present invention, of the present invention can be in forms such as tablets for medical, quasi drug, food and cosmetic purposes containing active ingredients. The forms also include rapidly disintegrating tablets made of the base which is produced by the production method of a base for a rapidly disintegrating tablet of the present invention.

The rapidly disintegrating tablet may be a tablet obtained by isothermally heating the compact at temperature equal to or above the glass transition temperature of the acrylic copolymer for a given period of time, an uncoated tablet (a plain tablet) of the tablet, or a tablet (a coated tablet or sugar-coated tablet) obtained by covering the uncoated tablet with, for example, a moisture protecting coat, an enteric coat or sugar coat. And the plain tablet is preferable.

Examples of the plain tablet include buccal tablets (such as troches), effervescent tablets, oral mucoadhesive tablets, intrabuccally disintegrating tablets and dry coated tablets. And the effervescent tablets and orally disintegrating tablets which quickly disintegrate with water or digestive fluid are preferable.

### - Rapid Disintegration Ability -

In the case of orally disintegrating tablets as an example of the rapidly disintegrating tablet can preferably disintegrate by a very small amount of water such as saliva in the oral cavity within 2 minutes, and more preferably 1 minute.

### - Strength -

The strength of the rapidly disintegrating tablet can generally be evaluated based on hardness and abrasion resistance which are measures used for evaluating tablets.

That hardness can be measured with, for example, HARDNESS TESTER (a hardness tester manufactured by Fujiwara Scientific Co., Ltd.). That abrasion resistance can be measured with, for example, Friabilator (manufactured by Toyama Sangyo Co., Ltd.).

The hardness of the rapidly disintegrating tablet is preferably 1.1 to 10 times higher than the hardness of the compact before the isothermal heating process, or before kept at a temperature in the range of 50°C to 100°C. The hardness is more preferably 1.1 to 8.0 times higher, and further preferably 1.2 to 5.0 times higher than the hardness of the compact before the isothermal heating process. Preferred specific value of hardness is, for example, in the range of 9N to 500N. It is more preferably in the range of 19N to 300N, and further preferably 29N to 196N.

Particularly, the hardness of the orally disintegrating tablet is preferably in the range of 9N to 200N, more preferably 19N to 150N, and further preferably 29N to 100N.

According to the present invention, pharmaceutical preparations can be provided. The preparations can easily be swallowed by patients having poor swallowing capability, while having capability of preventing reduction in its quality caused by, for example, breakage during distribution and storage or a dispensing process by a tablet packing machine and of maintaining high quality. Furthermore, according to the present invention, production steps can be simplified and productivity can be improved because a specialized machine for producing an orally disintegrating tablet is not required, and a common tableting machine can be used. And because the present invention can be applied regardless of sorts of drugs and additives (except aminoalkyl methacrylate copolymer E), it has general versatility as elemental technology. The production method of the present invention can also produce tablets when a drug is not formulated into the tablets, and therefore it can be applied to producing dosage forms of tablets for food and cosmetic applications.
(1) A production method of a tablet, including:
   (i) mixing a drug, aminoalkyl methacrylate copolymer E and at least a pharmaceutically acceptable additive to obtain a mixture thereof,
   (ii) tableting the mixture to obtain a compact, and (iii) isothermally heating the compact at a temperature in the range of from 50°C to 100°C.
(2) The production method according to (1), wherein
   the formulating amount of aminoalkyl methacrylate copolymer E is in the range of 0.5 parts by mass to 20 parts by mass per 100 parts by mass of the compact.
(3) The production method according to (1), wherein
   the tablet is an orally disintegrating tablet.
(4) The production method according to (3), wherein
   the formulating amount of aminoalkyl methacrylate copolymer E is in the range of 0.5 parts by mass to 9 parts by mass per 100 parts by mass of the compact.
(5) A production method of a compact, including:
   tableting a mixture including aminoalkyl methacrylate copolymer E and at least a pharmaceutically acceptable additive to obtain a compact of the mixture, and
   isothermally heating the compact at a temperature in the range of from 50°C to 100°C.
(6) The production method according to one of (1) and (5),
   wherein
   the average particle diameter of aminoalkyl methacrylate copolymer E in the mixture is in the range of 1µm to 500µm.
(7) A production method of a tablet, including:
   (a) mixing a drug, aminoalkyl methacrylate copolymer E and at least a pharmaceutically acceptable additive to obtain a mixture thereof, and
   (b) tableting the mixture to obtain a compact, and (c) isothermally heating the compact at a temperature in the range of from 50°C to 100°C.
(8) A tablet obtained based on a compact, wherein
   the compact has a drug, aminoalkyl methacrylate copolymer E and at least a pharmaceutically acceptable additive, and
   a part or the entire of aminoalkyl methacrylate copolymer E is cross-linked.
(9) A tablet obtained based on a compact, wherein
   the compact has a drug, aminoalkyl methacrylate copolymer E and at least a pharmaceutically acceptable additive,
   the hardness of the compact after isothermally heated at a temperature is 1.1 to 10 times higher than the hardness of the compact before isothermally heated, and
   the hardness of the compact after isothermally heated at the temperature is obtained by isothermally heating the compact at the temperature which is in the range of from 50°C to 100°C.
(10) The tablet according to one of (7) and (9), wherein
   the tablet is a plain tablet, an effervescent tablet or an orally disintegrating tablet.
(11) A compact which is obtained by tableting a mixture including aminoalkyl methacrylate copolymer E and at least a pharmaceutically acceptable additive to obtain a compact of the mixture, and isothermally heating the compact at a temperature in the range of from 50°C to 100°C.

### Examples

Although Examples of the present invention will be described below, it is understood that the present invention is not limited to Examples thereof.

Compounds used in Examples are compliant with official compendiums such as the Japanese Pharmacopoeia Fourteenth Edition (hereinafter it may be referred to as JP), the Japan Pharmaceutical Excipient Standards 2003 (hereinafter it may be referred to as JPE), the Japanese Pharmaceutical Codex 1997 (hereinafter it may be referred to as JPC) and the Japanese Standards of Food Additives (hereinafter it may be referred to as JSFA).

### Example 1

A crystalline cellulose (tradename: Avicel PH301 (hereinafter it may be referred to as Avicel PH301), manufactured by Asahi Chemical Industry Co., Ltd.) weighing 115.36g and mannitol (tradename: Parteck M200, manufactured by Merck Ltd.) weighing 173.04g were used as the additives, and aminoalkyl methacrylate copolymer E (tradename: EUDRAGIT (registered trademark) EPO, manufactured by Degussa) weighing 11.6g was used as an acrylic copolymer. They were mixed using MECHANOMILL (manufactured by OKADA SEIKO CO., LTD.) at 800 rpm for 3 minutes.

Portions of the thus obtained mixture were tableted under tableting pressures, 2.0kN, 2.5kN and 2.9kN, respectively, with targeting weight of 180mg. Kikusui Small High Velocity Revolution Type Tablet-machine (VIRGO 0512SS2AZ) - External Lubricant Spray System (ELS-P1 Typel) were used for the tableting processes. At this time, a tableting die which was 8mm in inner diameter and a punch were used, magnesium stearate was used as an external lubricant. Thus, compacts weighing 180mg were obtained.

After kept at 80°C for 10 hours, the obtained compacts were allowed to stand and cool to room temperature. Thereby rapidly disintegrating tablets (orally disintegrating tablets) composed of only the base for rapidly disintegrating tablet were obtained.

### <Measurement of Disintegration Property in the Oral Cavity>

By having subjects take one of the obtained rapidly disintegrating tablets, the disintegration time in the oral cavity was measured. The disintegration time in the oral cavity shall mean a time which is from when a rapidly disintegrating tablet is contained in the oral cavity until the tablet disintegrates and loses its form naturally with saliva and without the movement of the tongue. Shorter disintegration time in the oral cavity means better disintegration property in the oral cavity.

As a result, the disintegration time in the oral cavity of the rapidly disintegrating tablet made under tableting pressure of 2.0kN was 0.23 minutes, 2.5kN was 0.32 minutes and 2.9kN was 0.63 minutes.

### <Measurement of Hardness>

The hardness of the obtained rapidly disintegrating tablets was measured using a tablet hardness tester (tradename: Hardnesstester, manufactured by Fujiwara Scientific Co., Ltd.). Additionally, for comparison, the hardness of the compacts was measured in the same manner before the compacts were given heat. The results are shown in FIG. 2.

From the results shown in FIG. 2, it was confirmed that the isothermal heating process can increase hardness. Additionally, it was suggested that an orally disintegrating tablet can be produced even under low tableting pressure, 2.0kN, and further an orally disintegrating tablet may be produced by tableting under tableting pressure lower than 2.0kN. Moreover, it was suggested that, by controlling tableting pressure, orally disintegrating tablets having targeted hardness and disintegration property can be obtained.

### Example 2

Amlodipine besilate (manufactured by Dr. Reddy's Laboratories Ltd.) weighing 0.35g was used as the agent, a crystalline cellulose (tradename: Avicel PH101 (hereinafter it may be refereed to as Avicel PH101), manufactured by Asahi Chemical Industry Co., Ltd.) weighing 6.9g and mannitol (manufactured by Towa Chemical Industry Co., Ltd.) weighing 10.4g were used as the additives, and aminoalkyl methacrylate copolymer E (tradename: EUDRAGIT (registered trademark) EPO, manufactured by Degussa) weighing 0.35g was used as the acrylic copolymer. They were sufficiently mixed in a polyethylene bag to thereby obtain a mixture thereof.

Then, the obtained mixture was tableted using Shimadzu Autograph AGS-1000D under tableting pressure of 2.9kN to obtain a compact weighing 180mg and having a diameter of 8mm. At the tableting process, surfaces of the tableting die and punch were covered with magnesium stearate (manufactured by Nippon Oil & Fats Co., Ltd.) as lubricant.

After kept at 80°C for 10 hours, the obtained compact was allowed to stand and cool to room temperature. Thereby a rapidly disintegrating tablet (orally disintegrating tablet) was obtained.

A disintegration time in the oral cavity and the hardness of the rapidly disintegrating tablet thus obtained were measured in the same manner as in Example 1. The result is shown in Table 1.

Additionally, a dissolution test of amlodipine besilate was conducted on the rapidly disintegrating tablets. The result is shown in FIG. 3.

### <Dissolution Test>

The result of the dissolution test was evaluated using the Second Method for Dissolution Test (Paddle method) issued by JP.

Dissolutiontester NTR-VS6P or NTR-6100 (manufactured by Toyama Sangyo Co., Ltd.) was used as a dissolution tester.

A liquid chromatography (Shimadzu Corporation 10A series, hereinafter it may be referred to as HPLC) was used for quantification of the drug in elute.

Test conditions were as follows:
Apparatus: Paddle method (50 rpm)
Test liquid: The Second Liquid for Disintegration Test (pH 6.8) issued by JP
Amount of test liquid: 900mL
Temperature of test liquid: 37°C (plus/minus 0.5°C)
Test length: 5,10, 15, 30 and 45 minutes

### - HPLC -

The conditions of the quantification by HPLC were as follows:
Detector: an ultraviolet absorptiometer (measurement wavelength: 240nm)
Column: InertsilODS - 24.6mmx15cm, 5µm
Column temperature: 40°C
Flow rate: 0.9 mL/min
Analysis time: 6 minutes
Injection amount: 40µL
Mobile phase for HPLC: Methanol and potassium dihydrogenphosphate (41->10,000) mixture (13:7) solution

### <Preparation of Standard Solution for Quantitative Determination>

Methanol was added to and dissolved in a reference standard weighing 38.5mg to obtain solution amounting to 100mL (0.385mg/mL). Two milliliters was taken from the solution, and methanol was added thereto to obtain solution amounting to 200mL (0.00385 mg/mL). The solution thus obtained shall be a base solution. Mixtures of the base solution and solvents for different dissolution tests (1:1) were prepared as standard solutions for each solution condition.

### <Preparation of Sample Solution>

Ten milliliters of elute was collected, and then that elute was filtrated through a filter (HLC-DISK25 water, pore size of 0.45µm, manufactured by KANTO CHEMICAL CO., INC.). At least the first 4ml of elute was discarded and then the subsequent elute was collected. Mixture of that collected filtered-elute and methanol (1:1) was prepared as sample solution.

### Example 3

Amlodipine besilate (manufactured by Dr. Reddy's Laboratories Ltd.) weighing 0.054g was used as the agent, Avicel PH101 (manufactured by Asahi Chemical Industry Co., Ltd.) weighing 1.13g and mannitol (manufactured by Towa Chemical Industry Co., Ltd.) weighing 1.70g were used as the additives, and aminoalkyl methacrylate copolymer E (tradename: EUDRAGIT (registered trademark) EPO, manufactured by Degussa) weighing 0.114g was used as the acrylic copolymer. They were sufficiently mixed in a polyethylene bag to thereby obtain a mixture thereof.

Then, the obtained mixture was tableted using Shimadzu Autograph AGS-1000D under tableting pressure of 2.9kN to obtain a compact weighing 180mg and having a diameter of 8mm. At the tableting process, surfaces of the tableting die and punch were covered with magnesium stearate (manufactured by Nippon Oil & Fats Co., Ltd.) as lubricant.

After kept at 80°C for 10 hours, the obtained compact was allowed to stand and cool to room temperature. Thereby a rapidly disintegrating tablet (orally disintegrating tablet) was obtained.

The disintegration time in the oral cavity and the hardness of the rapidly disintegrating tablet thus obtained were measured in the same manner as in Example 1. The result is shown in Table 1.

A dissolution test of amlodipine besilate was conducted in the same manner as in Example 2. The result is shown in FIG. 3. (Comparative Example 1)

Avicel PH301 (manufactured by Asahi Chemical Industry Co., Ltd.) weighing 120g and mannitol (tradename: Parteck M200, manufactured by Merck) weighing 180g were sufficiently mixed in a polyethylene bag to thereby obtain a mixture thereof. The thus obtained mixture was tableted under tableting pressure in the range of 4.2kN to 4.4kN. Kikusui Small High Velocity Revolution Type Tablet-machine (VIRGO 0512SS2AZ) - External Lubricant spray System (ELS-P1 Typel) were used for the tableting process. At this time, the tableting die which is 8mm in inner diameter and the punch were used, magnesium stearate was used as an external lubricant, and a compact weighing 180mg was obtained.

After kept at 80°C for 10 hours, the obtained compact was allowed to stand and cool to room temperature. Thereby a tablet composed of only the additive and free from the acrylic copolymer was obtained.

A disintegration time in the oral cavity and the hardness of the obtained tablet were measured in the same manner as in Example 1. The result is shown in Table 1.

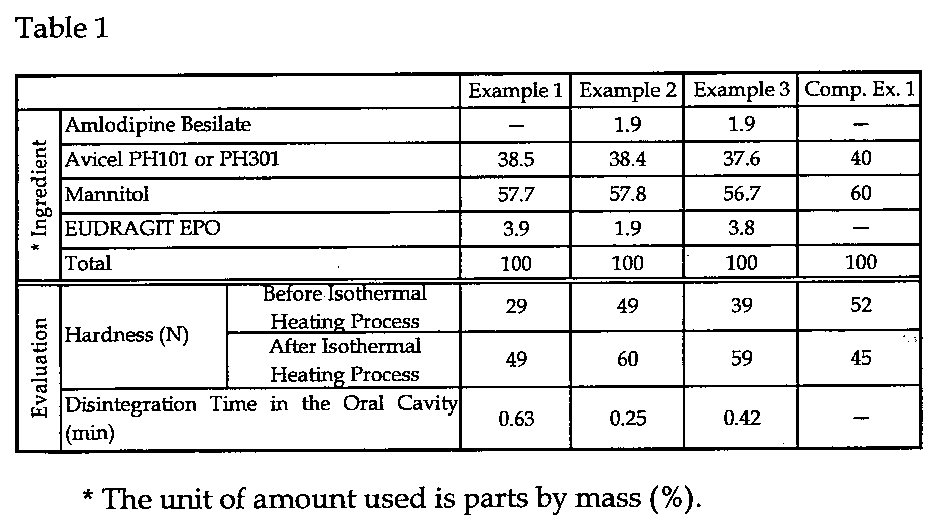

From the results shown in Table 1, it was confirmed that tablet strength was improved by formulating aminoalkyl methacrylate copolymer E as an acrylic copolymer and isothermally heating the compact at 80°C, a temperature equal to or above the glass transition temperature of the acrylic copolymer, for a given period of time. Furthermore, in the test of the disintegration property in the oral cavity, the tablet disintegrated quickly in the oral cavity, and no gritty feeling of the tablet on the tongue was recognized, and further, it was easily swallowed. From those results, it was suggested that rapid disintegration was achieved in the rapidly disintegrating tablet produced by the production method of the present invention, while maintaining its strength.

As the result of the dissolution test of amlodipine besilate shown in FIG. 3 shows, the rapidly disintegrating tablet of the present invention produced a good dissolution profile as a pharmaceutical composition.

From the above-mentioned results, it was confirmed that the component contributing to the characteristics of the rapidly disintegrating tablet of the present invention is acrylic copolymer (aminoalkyl methacrylate copolymer E).

That suggests that, in the pharmaceutical composition, the purpose of the present invention can be achieved regardless of sorts of formulated drugs and/ or other additives.

The present invention can also be used to tablets for food and cosmetic purposes in which drug is not formulated. Moreover, when aminoalkyl methacrylate copolymer E was formulated in the orally disintegrating tablet, the copolymer had effect even when its formulated amount was less than 10 parts by mass per 100 parts of the compact. And the cross-linked structure generated by isothermally heating the copolymer at a constant temperature had little effect on its disintegration and dissolution properties and easiness to take.

### (Example 4: Plain Tablet)

Amlodipine besilate (manufactured by Dr. Reddy's Laboratories Ltd.) weighing 0.054g was used as the active agent, Avicel PH101 (manufactured by Asahi Chemical Industry Co., Ltd.) weighing 1.07g and mannitol (manufactured by Towa Chemical Industry Co., Ltd.) weighing 1.61g were used as the additives, and aminoalkyl methacrylate copolymer E (tradename: EUDRAGIT (registered trademark) EPO, manufactured by Degussa) weighing 0.27g was used as the acrylic copolymer. They were sufficiently mixed in a polyethylene bag to thereby obtain a mixture thereof.

Then, the obtained mixture was tableted using Shimadzu Autograph AGS-1000D under tableting pressure of 2.9kN to obtain a compact weighing 180mg and having a diameter of 8mm. At the tableting process, surfaces of the tableting die and punch were covered with magnesium stearate (manufactured by Nippon Oil & Fats Co., Ltd.) as lubricant.

After kept at 80°C for 10 hours, the obtained compact was allowed to stand and cool to room temperature. Thereby a rapidly disintegrating tablet (plain tablet) was obtained.

The hardness of the thus obtained rapidly disintegrating tablet was measured in the same manner as in Example 1. The result is shown in Table 2.

### (Example 5: Plain Tablet)

Amlodipine besilate (manufactured by Dr. Reddy's Laboratories Ltd.) weighing 0.175g was used as the agent, Avicel PH101 (manufactured by Asahi Chemical Industry Co., Ltd.) weighing 3.46g and mannitol (manufactured by Towa Chemical Industry Co., Ltd.) weighing 5.19g were used as the additives, and aminoalkyl methacrylate copolymer E (tradename: EUDRAGIT (registered trademark) EPO, manufactured by Degussa) weighing 1.775g was used as the acrylic copolymer. They were sufficiently mixed in a polyethylene bag to thereby obtain a mixture thereof.

Then, the obtained mixture was tableted using Shimadzu Autograph AGS-1000D under tableting pressure of 2.9kN to obtain a compact weighing 180mg and having a diameter of 8mm. At the tableting process, surfaces of the tableting die and punch were covered with magnesium stearate (manufactured by Nippon Oil & Fats Co., Ltd.) as lubricant.

After kept at 80°C for 10 hours, the obtained compact was allowed to stand and cool to room temperature. Thereby a rapidly disintegrating tablet (plain tablet) was obtained.

The hardness of the thus obtained rapidly disintegrating tablet was measured in the same manner as in Example 1. The result is shown in FIG. 2.

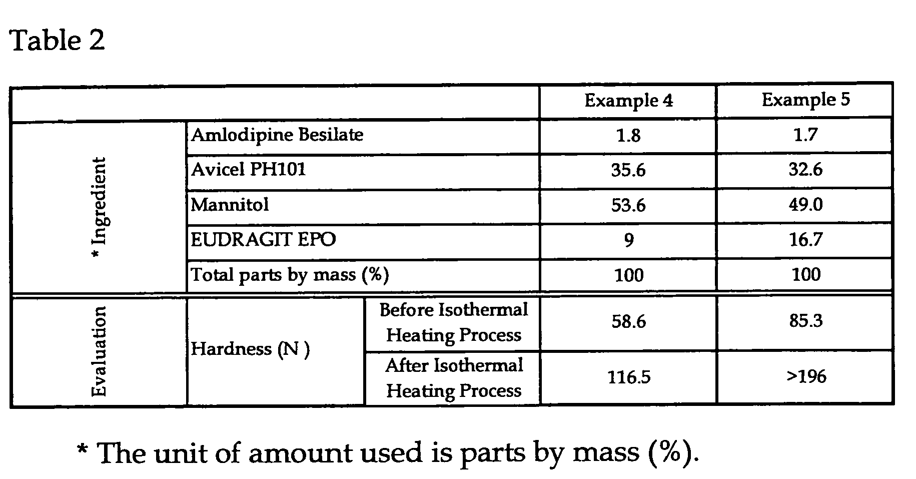

From the results shown in Table 2, it was confirmed that the strength of the obtained tablet was improved by formulating aminoalkyl methacrylate copolymer E into the tablet and isothermally heating it at a temperature equal to or above its glass transition temperature.

### (Example 6: Orally Disintegrating Tablet)

Amlodipine besilate (manufactured by Dr. Reddy's Laboratories Ltd.) weighing 11.6g was used as the agent, Avicel PH101 (manufactured by Asahi Chemical Industry Co., Ltd.) weighing 226g and mannitol (tradename: Parteck M200, manufactured by Merck) weighing 339g were used as the additives, and aminoalkyl methacrylate copolymer E (tradename: EUDRAGIT (registered trademark) EPO, manufactured by Degussa) weighing 23.2g was used as the acrylic copolymer. They were mixed using MECHANOMILL (manufactured by OKADA SEIKO CO., LTD.) for 3 minutes at 800 rpm to thereby obtain a mixture thereof.

The thus obtained mixture was tableted with targeting weight of 180mg. Kikusui Small High Velocity Revolution Type Tablet-machine (VIRGO 0512SS2AZ) - External Lubricant Spray System (ELS-P1 Typel) were used for the tableting processes. At this time, the tableting die which is 8mm in inner diameter and the punch were used, magnesium stearate was used as an external lubricant, and a compact weighing 180mg was obtained.

After kept at 80°C for 10 hours, the obtained compact was allowed to stand and cool to room temperature. Thereby a rapidly disintegrating tablet (orally disintegrating tablet) was obtained.

The hardness and disintegration property in the oral cavity of the rapidly disintegrating tablet (orally disintegrating table) were measured in the same manner as in Example 1.

As a result, while the hardness of the compact before the isothermal heating process was 45N, the hardness of the orally disintegrating tablet obtained was 100N. Additionally, the disintegration time in the oral cavity was 0.45 minutes.

And storage stability was measured as follows. The result is shown in Table 3.

### <Storage Stability>

The rapidly disintegrating tablet (orally disintegrating tablet) obtained in Example 6 was put into a clear glass bottle (hereinafter, the term "close" shall mean the bottle is hermetically-closed and "open" shall mean the bottle is opened) used as a preservation container, and the bottle was kept for four weeks in a constant temperature bath (tradename: DN94, manufactured by YAMATO SCIENTIFIC CO., LTD.) at 60°C.

Before and after the tablet was kept in the bottle, the hardness and disintegration time in the oral cavity of the tablet were measured in the same manner as in Example 1. The result is shown in Table 3.

**Table 3**

| Storage Condition | Storage Length | Hardness (N) | Disintegration Time in the Oral Cavity (min) |
|---|---|---|---|
| Initial value | Before Storage | 97 | 0.45 |
| 60°C (open) | 4 weeks | 104 | 0.52 |
| 60°C (close) | 4 weeks | 77 | 0.40 |

As the results shown in Table 3 indicate, change in the appearance of the tablet was not observed even when it was kept at a temperature equal to or above the glass transition temperature (50°C) of the acrylic copolymer. And further, its hardness and disintegration time in the oral cavity were little affected. Thus, it was confirmed that the tablet can maintain its quality as an orally disintegrating table.

### Example 7

Rapidly disintegrating tablets were obtained for each isothermal holding time of the isothermal heating process in Example 6, where respective isothermal holding time was 1, 3, 5 and 10 hours. Hardness was measured and a dissolution test (pH6.8) was conducted for each rapidly disintegrating tablet obtained in the same manner as in Example 1.

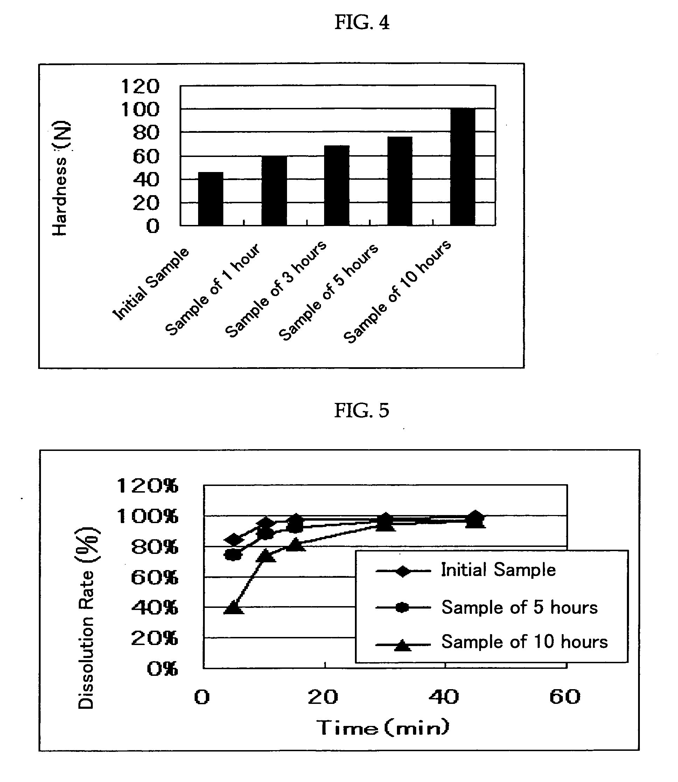

Additionally, for comparison, compacts before the isothermal heating process were evaluated in the same manner (hereinafter, they may be referred to as an Initial Sample) as in Example 1. Measured values of hardness are shown in FIG. 4, and the results of the dissolution tests are shown in FIG. 5.

From the results shown in FIG. 4, it was confirmed that longer isothermal holding time can increase hardness. The results indicats that strength can be improved by isothermally heating the compacts in which the acrylic copolymer (aminoalkyl methacrylate copolymer E) is formulated, and further, that strength can be controlled by changing the isothermal holding time.

On the other hand, from the results shown in FIG. 5, it was confirmed that both the Initial Sample (compacts) which was not isothermally heated and the rapidly disintegrating tablets after the isothermal heating process of the present invention elute almost completely within 45 minutes.

Moreover, the disintegration time in the oral cavity of a sample which was kept at a constant temperature with isothermal holding time of 10 hours was 0.45 minutes, thus it was confirmed that quality of the pharmaceutical composition can be maintained after the isothermal heating process in the present invention.

### (Examples 8 to 10 and Comparative Examples 2 to 3)

Mixtures were obtained by mixing raw materials with the compositions shown in the following Table 4. They were sufficiently mixed in polyethylene bags.

Then, the obtained mixtures were tableted using Shimadzu Autograph AGS-1000D under tableting pressure of 2.9kN to obtain compacts weighing 180mg per one tablet and having a diameter of 8mm. At the tableting process, surfaces of the tableting die and punch were covered with magnesium stearate (manufactured by Nippon Oil & Fats Co., Ltd.) as lubricant.

After kept in the constant temperature bath at 80°C for 10 hours, the compacts were allowed to stand and cool to room temperature. Thereby tablets were obtained.

In addition, the glass transition point (Tg) of EUDRAGIT RS was about 55°C. And the melting point of PEG 6000 which was formulated instead of the acrylic copolymer in Comparative Examples 2 and 3 was in the range of from 56°C to 61°C.

**Table 4**

| | Example 8 | | Example 9 | | Example 10 | | Comp. Ex. 2 | | Comp. Ex. 3 | |
|---|---|---|---|---|---|---|---|---|---|---|
| | parts by mass (%) | Batch Scale (mg) | parts by mass (%) | Batch Scale (mg) | parts by mass (%) | Batch Scale (mg) | parts by mass (%) | Batch Scale (mg) | parts by mass (%) | Batch Scale (mg) |
| Avicel PH101 | 39 | 706 | 39 | 706 | 37.7 | 706 | 39 | 706 | 37.7 | 706 |
| Mannitol | 59 | 1058 | 59 | 1058 | 56.5 | 1058 | 59 | 1058 | 56.5 | 1058 |
| EUDRAGIT EPO | 2 | 36 | - | - | - | - | - | - | - | - |
| EUDRAGIT RS | - | - | 2 | 36 | 5.8 | 108 | - | - | - | - |
| PEG 6000 | - | - | - | - | - | - | 2 | 36 | 5.8 | 108 |
| TOTAL | 100 | 1800 | 100 | 1800 | 100 | 1872 | 100 | 1800 | 100 | 1872 |

### <Evaluation of Hardness>

The hardness and disintegration property in the oral cavity of obtained tablets were measured in the same manner as Example 1. Two tablets of each condition were measured. The results (average values) are shown in Table 5.

**Table 5**

| | Hardness (N) | | | Disintegration Time in the Oral Cavity (sec) |
|---|---|---|---|---|
| | Before Isothermal Heating Process (Compact) | After Isothermal Heating Process (Tablet) | Hardness Ratio (Tablet / Compact) | |
| Ex.8 | 41.2 | 84.5 | 2.1 | 42.9 |
| Ex.9 | 35.3 | 35.3 | 1.0 | 15.2 |
| Ex.10 | 33.8 | 36.5 | 1.1 | 120 |
| Comp. Ex. 2 | 37.7 | 23.5 | 0.003 | 16.3 |
| Comp. Ex. 3 | 40.9 | 17.4 | 0.43 | 45.8 |

As the results shown in Table 5 indicate, in Example 8 in which 2 parts by mass of EUDRAGIT EPO was formulated as an acrylic copolymer, an excellent rapidly disintegrating tablet having oral disintegration property in the oral cavity while maintaining its strength was obtained. The rapidly disintegrating tablets which were obtained in Examples 9 and 10 using EUDRAGIT RS as an acrylic copolymer were excellent in disintegration property in the oral cavity, but the hardness thereof little changed after the isothermal heating process and they were somewhat weak in their strength.

On the other hand, the isothermal heating processes weakened hardness in Comparative Examples 2 and 3 in which PEG 6000 was used, and the compositions thus obtained could not function as tablets.

### <Evaluation of Storage Stability>

The tablets were isothermally heated in a constant temperature bath at 25°C and 75% relative humidity for 2 weeks. And then the hardness of the tablets was measured in the same manner as in Example 1. The result is shown in Table 6. For measuring hardness, a KIYA-type digital hardness tester, KHT-20 (measuring range of 0.5kgf to 20kgf) was used.

**Table 6**

| | Hardness (N) | | Hardness Ratio (Before / After Isothermal Heating) |
|---|---|---|---|
| | Before | After | |
| Ex. 8 | 84.5 | 50.5 | 0.597 |
| Ex. 9 | 35.3 | 17.2 | 0.487 |
| Ex. 10 | 36.5 | 17.2 | 0.471 |
| Comp. Ex. 2 | 23.5 | 6.6 | 0.021 |
| Comp. Ex. 3 | 17.4 | <4.9 | <0.282 |

As the results shown in Table 6 show, the reduction in the hardness of the rapidly disintegrating tablets of Examples 8 to 10 was smaller than that of Comparative Examples 2 and 3. The tablets of Example 8 to 10 maintained rapid disintegration ability while showed excellent storage stability.

### Industrial Applicability

The production method of a rapidly disintegrating tablet, the method that has versatility and, without a specialized device and with a normal tableting machine, can efficiently produce a rapidly disintegrating tablet having a desired rapid disintegration property and sufficient strength. Thus, the production method is preferable for producing rapidly disintegrating tablets for pharmaceutical preparation and quasi drug purposes, the tablets that can be easily swallowed by patients having a poor swallowing capability and have sufficient strength so that they can endure breakage and abrasion in processes such as distribution and storage and a dispensing process by a tablet packing machine. The production method is also preferable for producing rapidly disintegrating tablets for food (such as functional food and confectionery) and cosmetic purposes.

Furthermore, the production method of a base for a rapidly disintegrating tablet, the method that has versatility and, without a specialized device and with a normal tableting machine, can efficiently produce a rapidly disintegrating tablet having a desired rapid disintegration property and sufficient strength. Thus the production method is preferable for producing the base for rapidly disintegrating tablets that have excellent strength so that they can endure breakage and abrasion in processes such as distribution and storage and a dispensing process by a tablet packing machine.

## Claims

1. A production method of a rapidly disintegrating tablet, comprising:
(1) mixing an active ingredient, an acrylic copolymer and at least a pharmaceutically acceptable additive to obtain a mixture thereof,
(2) tableting the mixture to obtain a compact of the mixture, and
(3) isothermally heating the compact at a temperature in the range of from 50°C to 100°C for a given period of time.

2. The production method according to claim 1, wherein the active ingredient is a medicinal ingredient.

3. The production method according to one of claims 1 and 2, wherein the acrylic copolymer is composed of a methyl methacrylate, a butyl methacrylate and a dimethylaminoethyl methacrylate.

4. The production method according to any one of claims 1 to 3, wherein the acrylic copolymer is aminoalkyl methacrylate copolymer E.

5. The production method according to any one of claims 1 to 4, wherein the formulating amount of the acrylic copolymer is in the range of 0.5 parts by mass to 20 parts by mass per 100 parts by mass of the compact.

6. The production method according to any one of claims 1 to 5, wherein the formulating amount of the acrylic copolymer is in the range of 2 parts by mass to 4 parts by mass per 100 parts by mass of the compact.

7. The production method according to any one of claims 1 to 6, wherein the average particle diameter of the acrylic copolymer is in the range of 1µm to 500µm.

8. A production method of a base for a rapidly disintegrating tablet, comprising:
(1) mixing an acrylic copolymer and at least a pharmaceutically acceptable additive to obtain a mixture thereof,
(2) tableting the mixture to obtain a compact of the mixture, and
(3) isothermally heating the compact at a temperature in the range of from 50°C to 100°C for a given period of time.

9. A rapidly disintegrating tablet produced by the production method according to any one of claims 1 to 7.

10. A rapidly disintegrating tablet comprising the base produced by the production method according to claim 8.

11. The rapidly disintegrating tablet according to one of claims 9 and 10, wherein the hardness of the rapidly disintegrating tablet is 1.1 to 10 times higher than the hardness of the compact that is to be isothermally heated at a temperature in the range of 50°C to 100°C.

12. The rapidly disintegrating tablet according to any one of claims 9 to 11, wherein the rapidly disintegrating tablet is a plain tablet, an effervescent tablet or an orally disintegrating tablet.
